# EUROPEAN PATENT APPLICATION

(11) **EP 1 634 540 A1**
(43) Date of publication of application: **15.03.2006**
(21) Application number: 05077004.9
(22) Date of filing: 02.09.2005
(51) Int. Cl.: A61B 18/14, A61B 17/122, A61B 17/12, A61B 17/28

(54) **Surgical clamp and cutting blade**

(30) Priority: 08.09.2004 US 935822
(71) Applicant: Thompson Surgical Instruments, Inc., Traverse City MI 49684 (US)
(72) Inventor: Drake, Daniel H, Traverse City, Michigan 49684 (US); Mulac, Antony J, Traverse City, Michigan 49684 (US); Farley, Daniel K, Traverse City, Michigan 49684 (US)
(74) Representative: Hitchcock, Esmond Antony

(57) **Abstract**

A surgical clamp includes a first member (20) having a first handle (32), a first jaw (30), and a first pivot point intermediate the first handle and the first jaw. A second handle member (22) has a second handle (52), a second jaw (50), and a second pivot point intermediate the second handle and the second jaw. The second handle member is pivotally connected (66) to the first handle member at their respective pivot points. The first and second jaws cooperate to define a clamping element and a cutting element (100).

## Description

The present invention relates to a surgical clamp and cutting device. More particularly, the present invention relates to a surgical clamp and cutting device for use in connection with a wire sternotomy suture.

A sternotomy is an incision into or through the sternum and is typically part of the open heart surgery procedure. During such open heart surgery, the sternum is split to provide access to the inside of the patient's chest. After the surgery, the patient's sternum must be sutured back together. Typically, the sternum is sutured back together by passing a needle attached to a stainless steel wire through either side of the sternum, drawing the opposed sides of the sternum together so that the sternum may heal properly. In such a suturing procedure, it is typical to use a needle to lace the stainless steel wire through the sternum such that when the stainless steel wire is fully laced, the needle end of the wire and the tail end of the wire can be joined and pulled collectively to close the opposed sides of the sternum together. The typical stainless steel wire used in such suturing procedures has a diameter of about .75 to about 1 millimeter; typically several such wires are used to close the sternum.

Once the opposed sides of the sternum have been laced, the distal end and needle end of the wire are clamped or otherwise joined and the needle is cut off as close to the clamp as possible. Typically, the resulting short section of wire is very sharp. The clamps are used to grasp the wire ends to draw the opposed sections of the sternum back together and to twist tie the wire to firmly hold the sternum in place to enable the sternum to heal. The short section of wire protruding from the needle end is sharp and may often times tear the surgeon's gloves, actually cut the surgeon's hands, or cause damage to the patient if not effectively addressed.

Typically, a surgeon will use a standard surgical clamp to grasp the ends of the stainless steel wire. The surgeon may then use this same standard surgical clamp to twist the wires together. Finally, the surgeon will typically use a different cutting instrument to cut off the excess portions of the stainless steel wire.

Accordingly, it is one object of the present invention to provide a tool that combines in one device (1) the cutting functions necessary with the needle end of the stainless steal suturing wires and (2) the clamping function necessary for grasping and twisting of the stainless steel suturing wires.

A further object of the present invention is to provide a surgical tool that provides for an accurate cut of the stainless steel wire upon clamping and twisting of the wire.

These and other desirable characteristics of the present invention will become apparent in view of the present specification, including the claims and drawings.

The present invention is directed to a surgical clamp and cutting device, and more particularly, to a surgical clamp for use in grasping stainless steel wire sutures typically used to repair the sternum after open heart surgery. The surgical clamp and cutting device of the present invention further includes a cutting feature for cutting the stainless steel wire suture at a designated and beneficial location for eliminating excess stainless steel wire protrusions.

The surgical clamp and cutting device of the present invention includes two members pivotally attached adjacent one end of each member in typical pliers fashion. Each member has at one end a jaw. At the opposite end of each member is a handle that includes locking components such that when the two handles of the surgical clamp and cutting device are biased together, the locking components engage to lock both handles in a relatively compressed position, thereby biasing the jaws of the members together. Also, each handle includes a spring component for biasing the handles away from each other such that when the locking components are not engaged, the handles are separated by the spring components, thereby separating the jaws as well.

The jaw of each segment includes an element that when biased together with the element of the opposed jaw will cooperate to grip the stainless steel wire such that it may be firmly held, pulled, and twisted, and cut the wire at the desired predetermined and beneficial location. In a preferred embodiment, the elements constitute a set of teeth that are offset from one another, thereby bending the wire, as well as biting into the wire, for a firm grip of the stainless steel wire. Additionally, in a preferred embodiment, each of the elements includes a tooth which when biased together cut from both sides of the stainless steel wire. The elements enclose the cut end of the wire, thus protecting the surgeon. Of course, alternate element embodiments are contemplated, several of which are described below. The elements may be integral with the jaws of the surgical clamp and cutting device, however, it is preferred that each jaw include a slot such that the elements may be removably inserted in the slots and may be replaced at the end of their useful life. In this way, only the elements need be replaced instead of the entire device. The surgical clamp and cutting device of the present invention is preferably made of a material typically used for surgical instruments, such as stainless steel. The elements may also be made of such materials, such as stainless steel and preferably tungsten carbide.
Further features and advantages of the invention will be apparent from the following description of embodiments thereof, given by way of example. Reference will be made to the accompanying drawings wherein:

Figure 1 is an elevated, partial cross-section view of a surgical clamp;

Figure 2 is an elevated end view of one embodiment of the cutting element for use with a surgical clamp;

Figure 3 is an elevated end view of another embodiment of the cutting element for use with a surgical clamp;

Figure 4 is an elevated end view of yet another embodiment of the cutting element for use with a surgical clamp; and

Figure 5 is an elevated perspective view of the jaws of a clamp.

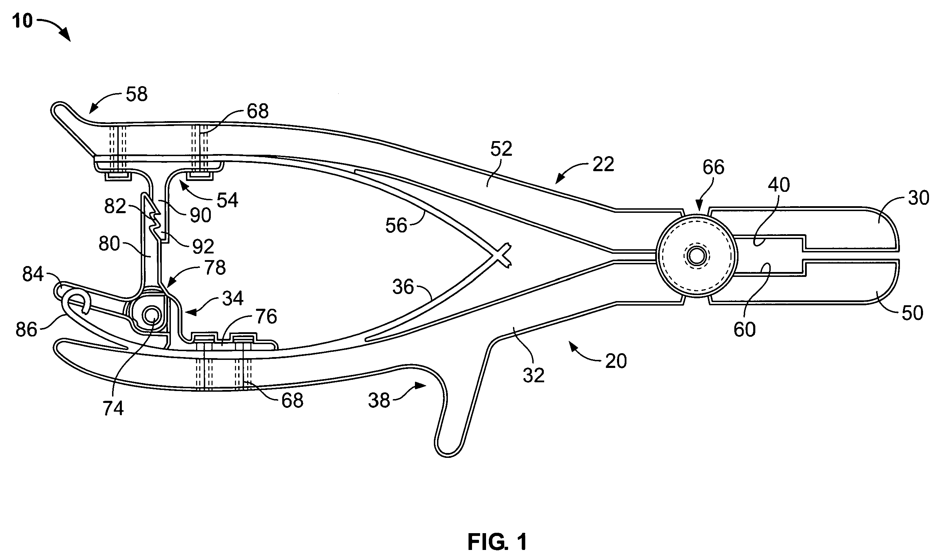

Figure 1 is an elevated, partial cross-sectional view of a surgical clamp 10 of the present invention. The surgical clamp 10 has a first member 20 and a second member 22.

The first member 20 includes a first member jaw 30, a first member handle 32, a first member locking component 34, a first member spring component 36, a first member hand seat 38, and a first member slot 40 positioned within the jaw 30. The second member 22 includes a second member jaw 50, a second member handle 52, a second member locking component 54, a second member spring component 56, a second member little, finger seat 58, and a second member slot 60 located within the second member jaw 50.

The first member 20 and the second member 22 are pivotally connected at pivot 66 in a manner conventionally used for prior art surgical clamps. The locking component 34 and the spring component 36 are fixedly attached to the handle 32 of the first member 20 by conventional screws 68. Handle 32 will have apertures adapted to receive conventional screws 68 for secure attachment of locking component 34 and spring component 36. As understood by those skilled in the art, any other conventional methods of fixedly attaching locking component 34 and spring component 36 to handle 32 may be employed. Also as understood by those skilled in the art, locking components 54 and 34 may be integral features of handles 22 and 20. Similarly, locking component 54 and spring component 56 are fixedly attached to the handle 52 of the second member 22. Spring component 56 and locking component 54 are fixedly attached to handle 52 with conventional screws 68. Handle 52 is adapted to receive conventional screws 68 in such a manner that locking component 54 and spring component 56 are securely attached to handle 52.

As can be seen in Figure 1, first member spring component 36 cooperates with second member spring component 56 in a conventional manner to bias first member handle 32 away from second member handle 52. This spring mechanism provides the outward force often employed in a surgical clamp or pliers generally. Those skilled in the art will readily recognize that any other conventional spring separating mechanism may be similarly employed to provide the outward force separating handle 32 and handle 52.

As further shown in Figure 1, first member locking component 34 cooperates with second member locking component 54 to maintain handle 32 and handle 52 in a locked position in opposition to the forces created by the cooperating first member spring component 36 and second member spring component 56. The first member locking component 34 includes a base 76 and a latch 78. The base 76 and latch 78 are pivotally connected by pivot pin 74. The latch 78 includes a spring seat 84 and a locking leg 80 that has locking teeth 82. The spring seat 84 extends away from the pivot point between the base 76 and the latch 78 providing a seat for the distal end 86 of the first member spring component 36.

Locking component 54 of the second member 22 also includes a locking leg 90 with locking teeth 92. When the handles 32 and 52 are compressed, locking teeth 82 of locking component 34 engage and cooperate with locking teeth 92 of locking component 54 to hold handle 32 and handle 52 in a locked position. The cooperation between distal end 86 of the spring component 36 and spring seat 84 of the latch 78 biases locking leg 80 of the locking component 34 towards locking leg 90 of the locking component 54 such that when the handles 32 and 52 are compressed the locking legs 80, 90 engage and cannot disengage without further interaction. In order to disengage locking leg 80 from locking leg 90, spring seat 84 of the latch 78 must be rotated away from locking leg 90 of locking component 54, thereby also rotating locking leg 80 away from locking leg 90. Although the locking mechanism as described herein is the preferred locking mechanism for use with the present invention, those skilled in the art will readily recognize the application of other conventional locking mechanisms that may also be suitably employed in the present invention.

When handle 32 and handle 52 are compressed such that locking leg 80 engages locking leg 90, jaw 30 and jaw 50 are similarly closed by virtue of the pivoting arrangement between the first member 20 and the second member 22. Naturally, when the locking legs 80 and 90 are disengaged permitting handle 32 and handle 52 to separate as a result of the cooperating spring force of spring components 36 and 56, jaw 30 and jaw 50 similarly separate.

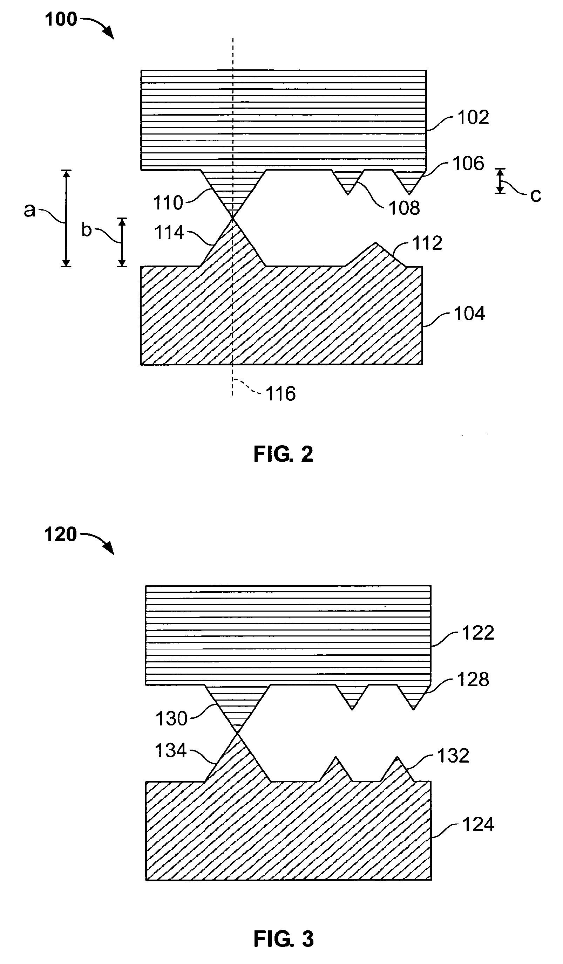

As noted above, jaw 30 includes slot 40 and jaw 50 includes slot 60 into which elements (Figures 2-4) may be inserted. It is the elements that grip and cut the stainless steel wire suture.

The surgical clamp 10 of the present invention is ergonomically designed such that the user must position the surgical clamp 10 in the correct orientation within the hand for use. Specifically, handle 32 of the first member 20 includes a hand seat 38 which is configured to be positioned between the thumb and index finger of the user while the handle 52 includes a little finger seat 58 to provide a positioning ledge for the user's little finger. In this way, the clamp is certain to be used in the correct orientation by the user. Another possibility to ensure correct orientation is to utilize indents for the fingers on the handles.

Figure 2 is an elevated end view of one embodiment of the cutting element 100 of the present invention. The cutting element 100 consists of an upper element or grip 102 and a lower element or pull 104. The grip 102 is adapted to fit within slot 40 of the jaw 30 (Figure 1) of the first member 20. The pull 104 is adapted to fit within slot 60 of the jaw 50 (Figure 1) of the second member 22. In the embodiment shown in Figure 2, the grip 102 includes a first tooth 106, a second tooth 108 and a cutting blade 110. The pull 104 includes a third tooth 112 and a cutting blade 114. As noted above, Figure 2 is an end view of the element 100. Thus, blade 110 and blade 114 run longitudinally along grip 102 and pull 104, respectively. In other words, the plane 116 passing vertically through the middle of blade 110 and the middle of blade 114 as shown in Figure 2 is generally parallel to the plane defined by first member 20 and second member 22 (Figure 1).

Similarly, teeth 106, 108 run longitudinally along inner surface of the grip 102 and tooth 112 runs longitudinally along the inner surface of the pull 104.

When the handle 32 and the handle 52 of the surgical clamp 10 are compressed, thereby closing jaws 30 and 50, the grip 102 and pull 104 are similarly compressed being located within the slot 40 and slot 60 (Figure 1), respectively. The action of simultaneously cutting and clamping the stainless steel wire suture demands that the jaws of a surgical clamp be designed to hold the stainless steel wire suture forcefully but not require so much pressure that it is difficult to cut the wire suture. The cutting element 100, as illustrated in Figure 2, accomplishes this goal by utilizing, in a preferred embodiment, a clamping means that has teeth that are offset from one another, thereby bending the wire as well as biting into the wire. Specifically, when the wire is placed between the grip 102 and the pull 102 and the jaws 30, 50 are closed, the third tooth 112 is generally disposed between the first tooth 106 and the second tooth 108, thereby bending the wire suture into a slight "S" shape providing for a secure grip on the wire suture. Simultaneously cutting blade 110 cooperates with cutting blade 114 to cut the wire suture at a pre-determined and beneficial distance from second tooth 108. In this way, the sharp cut end of the wire is contained within the grip and pull elements.

As noted above, the standard diameter of the stainless steel wire suture commonly used in typical surgical applications is about .75 to about 1 millimeter. For typical wires, one configuration of the element 100 may be a distance of about 1.2 mm (dimension a) between the inner surfaces of the grip 102 and pull 104, the height of each blades 110, 114 may be about 0.6 mm (dimension b), and the height of each of teeth 106, 108, 112 may be about 0.4 mm (dimension c).

As will be understood by those skilled in the art, the teeth 106, 108 and cutting blade 110 of the grip may be integrally formed with the jaw 30 of the first member 20 (Figure 1), and similarly the third tooth 112 and cutting blade 114 of the pull 104 may be formed integrally with the jaw 50 of the second member 22 (Figure 1). It is preferred, however, to provide the jaw 30 with slot 40 and jaw 50 with slot 60 to accommodate the cutting element 100 so that when the cutting element 100 has reached the end of its useful life, only the cutting element 100 need be replaced. The cutting element 100 may be made of materials typically used for surgical instruments such as stainless steel. The preferred material for the cutting element 100 is tungsten carbide.

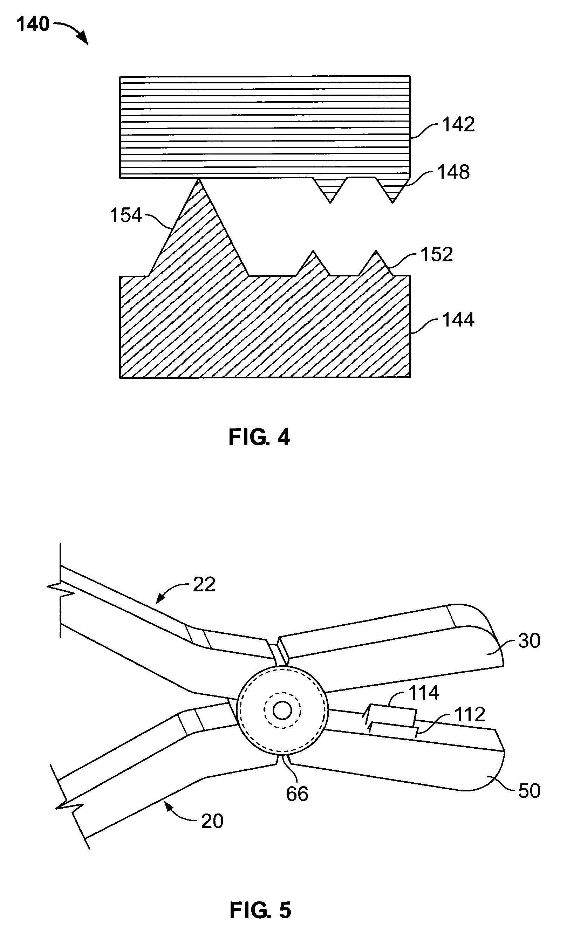

A better understanding of the operation of the surgical clamp 10 may be had by reference to Figure 5. The longitudinal alignment of the teeth and blades of the cutting element 100 can be seen in Figure 5 which is an elevated perspective view of the surgical clamp 10 in an opened position. As described above, the needle end of the stainless steel wire suture passes through the jaws 30, 50 such that the needle exits the area between the jaws at the blade 114 side of the surgical clamp 10. When the handles 20, 22 are biased together, jaws 30, 50 are also biased together bringing the teeth 106, 108 of the grip 102 into cooperating position with tooth 112. Simultaneously, blade 110 is brought into cooperating position with blade 114 to cut the needle from the stainless steel wire suture leaving the wire suture clamped by the cooperating clamping force of teeth 106, 108 of the grip 102 and tooth 112 of the pull 104.

Figure 3 is an elevated end view of another embodiment of the cutting element 120 of the present invention. The cutting element 120 includes a grip 122 and a pull 124. The grip 122 includes teeth 128 and cutting blade 130. The pull 124 includes teeth 132 and cutting blade 124. The cutting element 120, as shown in Figure 3, provides an alternative gripping arrangement to that shown in Figure 2.

Figure 4 is an elevated end view of another embodiment of the cutting element 140 of the present invention. In this embodiment, the grip 142 includes teeth 148. The pull 144 includes teeth 152. In this embodiment, only one of the elements includes a blade. Specifically, the pull 144 includes blade 154. Alternatively, the blade may be deployed on grip 142. Those skilled in the art will understand that the various teeth and blade arrangements disclosed can be interchanged to provide the desired configuration.

## Claims

1. A surgical clamp comprising:
a first member having a first handle, a first jaw, and a first pivot point intermediate the first handle and the first jaw;
a second handle member having a second handle, a second jaw, and a second pivot point intermediate the second handle and the second jaw; the second handle member being pivotally connected to the first handle member at their respective pivot points;
the first jaw and the second jaw cooperating to define a cutting element, the cutting element having at least a first tooth and at least a first blade; the first tooth being adapted to perform a grasping function, and the first blade being adapted to perform a cutting function.

2. A surgical clamp according to Claim 1 wherein the at least first tooth and the at least first blade are aligned longitudinally within the first jaw and the second jaw.

3. A surgical clamp according to Claim 2 wherein the cutting element comprises a first element and a second element, the first jaw comprises a first recess for accommodating the first element of the cutting element and the second jaw comprises a second recess for accommodating the second element of the cutting element, the first element being positioned within the first recess and the second element being positioned within the second recess.

4. A surgical clamp according to Claim 1 wherein the cutting element comprises a first element and a second element;
the first element having at least a first tooth and at least a first blade, and the second element having at least a second tooth and at least a second blade, and wherein the first tooth of the first element cooperates with the second tooth of the second element to perform a grasping function, and the first blade of the first element cooperates with the second blade of the second element and adapts to perform a cutting function.

5. A surgical clamp according to Claim 1 wherein the at least first tooth and the at least first blade of the first element are aligned longitudinally within the first jaw and the at least second tooth and the at least second blade of the second element are aligned longitudinally within the second jaw.

6. A surgical clamp according to Claim 5 wherein the first jaw comprises a recess for accommodating the first element of the cutting element and the second jaw comprises a recess for accommodating the second element of the cutting element.

7. A surgical clamp according to Claim 1 wherein the cutting element has a first element and a second element, the first jaw comprises a first recess and the second jaw comprises a second recess;
wherein the first element has at least a first tooth and said first blade and is fitted into the recess of the first jaw, the at least first tooth and the at least first blade being aligned longitudinally within the first jaw; and
wherein the second element has at least a second tooth and at least a second blade and is fitted into the second recess of the second jaw; the at least second tooth and the at least second blade being aligned longitudinally within the second jaw, whereby the first tooth of the first element cooperates with the second tooth of the second element to perform said grasping function, and the first blade of the first element cooperates with the second blade of the second element to perform said cutting function.

8. A surgical clamp according to any preceding Claim wherein:
the first handle comprises a first spring component and the second handle comprises a second spring component, the first spring component cooperating with the second spring component to bias the first handle away from the second handle; and
the first handle comprises a first locking component and the second handle comprises a second locking component, the first locking component cooperating with the second locking component and adapted to maintain the first handle and the second handle in a relatively closed position when the first locking component engages the second locking component.

9. A surgical clamp according to Claim 8 wherein the first spring component includes a distal end and the first locking component comprises a seat adapted for accommodating the distal end of the first spring component; whereby the distal end of the first spring component biases the first locking component into a position to engage the second locking component.
